# EUROPEAN PATENT SPECIFICATION

(11) **EP 3 573 532 B1**
(45) Date of publication and mention of the grant of the patent: **19.05.2021**
(21) Application number: 18703681.9
(22) Date of filing: 24.01.2018
(51) Int. Cl.: A61B 6/04, A61B 6/00, A61N 5/10

(54) **A UNIT AND EQUIPMENT FOR RADIOTHERAPEUTIC PRECLINICAL STUDIES**
EINHEIT UND AUSRÜSTUNG FÜR PRÄKLINISCHE STRAHLENTHERAPIESTUDIEN
UNITÉ ET ÉQUIPEMENT POUR ÉTUDES PRÉCLINIQUES RADIOTHÉRAPEUTIQUES

(30) Priority: 25.01.2017 CZ 20170039
(43) Date of publication of application: 04.12.2019
(73) Proprietor: Radalytica a.s., 77900 Holice, Olomouc (CZ)
(72) Inventor: UHER, Josef, 75661 Roznov Pod Radhostem (CZ); J W VERHAEGEN, Frank, 3770 Riemst (BE)
(74) Representative: Sedlák, Jirí
(86) International application number: PCT/CZ2018/050002
(87) International publication number: WO 2018/137727

(56) References cited:
- EP-A1- 3 106 093
- DE-A1-102014 207 568
- US-A1- 2011 222 667
- US-A1- 2016 338 656

## Description

### Field of the Invention

The invention concerns a unit and equipment for combined irradiation by a therapeutic particle beam and simultaneous irradiation by other ionizing radiation (e.g. photons) combined with the computed tomography (CT) imaging. The purposes are preclinical studies on animals using ionising radiation beams. The unit is placed inside an existing radiotherapy vault for human clinical radiotherapy by the therapeutic beam of particles.

### Background of the Invention

The so-called radiotherapy has been used recently in particular for the treatment of oncological diseases. A method of treatment where cancer tissue is destroyed by targeted irradiation by a therapeutic beam of ionizing radiation is concerned. The incident beam of the therapeutic ionizing radiation releases energy into the irradiated tissue that leads to irreversible damage to the tissue cells resulting in their destruction. Among the known kinds of therapeutic ionizing radiation are in particular photon radiation, e.g. x-ray radiation and a therapeutic beam of particles, such as protons or neutrons.

Another application of ionizing radiation is the acquisition of a three-dimensional model of the internal structure of the irradiated object by computed tomography. By the detection of the therapeutic beam of ionizing radiation passing through the irradiated object in different directions and by the analysis of changes in this beam due to transmission through the object, and also, for example, by the detection of e.g. fluorescent ionizing radiation or scattered ionizing radiation or back-scattered ionizing radiation, it is possible to acquire a three-dimensional model of the internal structure of the irradiated object or at least part of it. The aforementioned method utilizing computed tomography can be referred to as the CT scanning process.

Within the framework of radiotherapy both methods mentioned above are customarily used. The tissue intended for the applications of therapeutic ionizing radiation is localized and subsequently treated by radiotherapy. It is advantageous to perform both steps at the same time.

An example of such a solution is disclosed in the WO 2007060242 A1 document. The document deals with the equipment for the application of radiotherapy using a therapeutic particle beam and parallel imaging based on x-rays and computing tomography. Another similar solution is for example disclosed in the DE 102006000837 A1 patent application.

Both documents mentioned above present combined irradiation for therapeutic purposes and computed tomography. Also another device is known from document EP 3 106 093 A1 which allows the patient to be imaged during radiation therapy.

A drawback of photon radiation is that it affects also healthy tissue found along the path of its propagation. This drawback is partially compensated by therapeutic beams of particles that are able to release most of their energy at the end of their propagation path, the so-called Bragg peak. On the other hand, a disadvantage of therapeutic particle beams rests in the fact that currently there is not enough clinically ascertained information on the efficacy of the therapeutic beams of particles of certain types for specific kinds of tumour tissues.

Although there has been intensive research, the progress is limited by the number of laboratories and other sites allowing radiotherapeutic preclinical studies on animals to be performed.

Currently there is a very limited number of sites allowing radiotherapeutic preclinical studies using therapeutic particle beams, namely because of the fact that particle accelerators, whose purchase and operating costs are very high, are needed for the acquisition of the therapeutic particle beam. In a majority of cases, the clinical centres being built are equipped with radiotherapy vaults for clinical radiotherapy that earn money needed for the payment of operating and purchase costs by the treatment of patients. Therefore, such clinical centres are naturally interested in booking most of the operating time of such radiotherapy vaults for clinical treatment leaving only a minimum time for radiotherapeutic preclinical studies. Preclinical studies are usually performed on laboratory animals, such as rats and mice requiring equipment suitable for study-specific experiments on small irradiated objects. However, the existing space and equipment for radiotherapy vaults of clinical centres have been adapted for the treatment of large irradiated objects, and therefore it is necessary to adapt the existing equipment and space of the radiotherapy vault of the clinical centres for the preclinical studies.

Such adaptations would however reduce the capacity available for clinical treatment and are undesirable, which brings back again the urging problem of insufficient space for the performance of radiotherapeutic preclinical studies.

The task of the invention is the development of a unit and equipment for combined irradiation for preclinical studies that would be utilizable within the framework of the radiotherapy vaults of the existing clinical centres without requiring any radical adaptation of the radiotherapy vault of the clinical centre or change in the present clinical equipment. Moreover, the system allows all measurements and irradiation to be done without necessity of transferring the animal. This greatly improves the precision of measurement and irradiation.

### Summary of the Invention

The invention is defined in claim 1. Further aspects and preferred embodiments are defined in the dependent claims. Aspects, embodiments and examples of the present disclosure which do not fall under the scope of the appended claims do not form part of the claimed invention and are merely provided for illustrative purposes.

The set goal has been achieved by the development of a unit for the performance of radiotherapeutic preclinical studies on animals based on the following description.

The unit is intended for the irradiation of the object by a therapeutic particle beam, from an external source and parallel CT scanning of the object using at least one scanning pair, in particular for radiotherapeutic preclinical studies on animals. The scanning pair consists of the source of ionizing radiation and the imaging detector of ionizing radiation arranged on the opposite side.

The unit comprises a mobile platform equipped with an adjustable table supporting the irradiated object. In addition, the mobile platform is equipped with at least one load-bearing means that supports at least one scanning pair arranged in a movable manner as to the adjustable table. In addition, the load-bearing means, scanning pair and adjustable table delimit free working space for unshielded travel of the therapeutic particle beam through the unit.

According to the invention the mobile platform is equipped with at least one adjustable source of ionizing radiation. By adding a source of ionizing radiation, it is possible to perform combined irradiation including two types of therapeutic irradiation and one type of scanning irradiation at the same time.

The mobile platform allows an easy transport of the unit to its destination and also easy cleaning of the unit for the destination restoration. The mobile platform is equipped with instruments necessary for the execution of radiotherapeutic preclinical studies on animals, such as an adjustable table to place the irradiated object into the path of the therapeutic particle beam and load-bearing means to support the scanning pair that is movable as to the adjustable table to perform the scanning of the irradiated object from various directions within the framework of the CT scanning process. At the same time, the free working space, through which the therapeutic particle beam travels in an unshielded manner from the outlet of the external source of the therapeutic particle beam, is delimited. The therapeutic particle beam passes through the irradiated object placed on the adjustable table. Such arrangement is advantageous as it is not necessary to adjust the external source in any radical manner. All other equipment needed for radiotherapeutic preclinical studies is supplied to the site within the framework of the unit or cleaned from the site without limiting the function of the external source.

In a preferred embodiment of the unit according to the invention, the load-bearing means consists of a rotating support frame fixed using axial bearings between two columns protruding from the mobile platform. The rotating support column ensures the movability as to the adjustable table and the rotating support frame has at least two arms supporting the scanning pair allowing the arrangement of the scanning pair elements on the opposite sides. The columns and arms of the rotating support frame also delimit the free working space, and the inner rings of the axial bearings are arranged at the inlet of the therapeutic particle beam into the free working space and at the outlet of the therapeutic particle beam from the free working space not to shield the therapeutic particle beam travelling through the load-bearing means of the unit.

In a preferred embodiment of the unit according to the invention, the scanning pair on the arms of the rotating support frame is adjustable. Thanks to adjusting, the scanning pair can be adapted to various types of irradiated objects placed on the adjustable table.

In another preferred embodiment of the unit according to the invention, the load-bearing means consists of at least one robotic arm. The robotic arm is very well controllable and also very precise when moving as programmed. Robotic arms can work in an environment exposed to ionizing radiation.

Preferably, the source of therapeutic ionizing radiation is attached to the robotic arm itself to allow its location as to the irradiated tissue to be detected.

In a preferred embodiment of the unit according to the invention, the unit is equipped with at least one imaging detector of the therapeutic particle beam, preferably equipped with two imaging detectors of the therapeutic particle beam, where the first imaging detector is arranged at the inlet of the therapeutic particle beam into the delimited free working space and the other imaging detector is arranged at the outlet of the therapeutic particle beam from the free working space. By the monitoring of the shape and properties of the therapeutic particle beam prior to entering the irradiated tissue and by the subsequent monitoring of changes and consequences after the irradiation of the irradiated tissue, data for better understanding of the effect of the therapeutic particle beam on the irradiated tissue can be acquired. Preferably, the imaging detector is arranged at the rotating support frame or column or robotic arm itself.

The invention also includes equipment for the performance of radiotherapeutic preclinical studies on animals inside radiotherapy vaults for clinical radiotherapeutic irradiation.

The equipment includes radiotherapy vaults with the outlet of the external source of the therapeutic particle beam installed in the inner space of the vault.

The equipment comprises a unit designed according to any of the appended claims. The unit is placed in the inner space of the radiotherapy vault and in the equipment, it is oriented for unshielded passage of the therapeutic particle beam from the outlet of the therapeutic particle beam of the external source through the free working space delimited by the unit.

The equipment allows radiotherapeutic preclinical studies to be performed on animals using the existing radiotherapy vaults for the clinical radiotherapy of patients. Thanks to the equipment it is possible to perform preclinical studies without interventions in the equipment of radiotherapy vaults at the time when no therapy is delivered, and it is possible to restore the operation of the radiotherapy vault in a very short time to resume the treatment of patients.

Among the advantages of the invention is the possibility to perform preclinical studies to a large extent without having to build new radiotherapy vaults for preclinical studies requiring considerable expenses. The use of the existing radiotherapy vaults for the treatment of patients is temporary and does not interfere with the functionality for the treatment of patients.

The purchase costs of the unit are many times lower compared to those related to the construction of laboratories equipped with custom radiotherapy vaults for radiotherapeutic preclinical studies.

### Explanation of Drawings

The present invention will be explained in detail by means of the following figures where:
Fig. 1 illustrates the unit with a load-bearing means consisting of a rotating support frame,
Fig. 2 illustrates the unit with a load-bearing means consisting of robotic arms,
Fig. 3 illustrates a simplified image of the equipment for radiotherapeutic preclinical studies.

### An Example of the Invention Embodiment

It shall be understood that the specific cases of the embodiments described and depicted below are provided for illustration only and do not limit the invention to the examples provided here. The invention is defined in the appended claims.

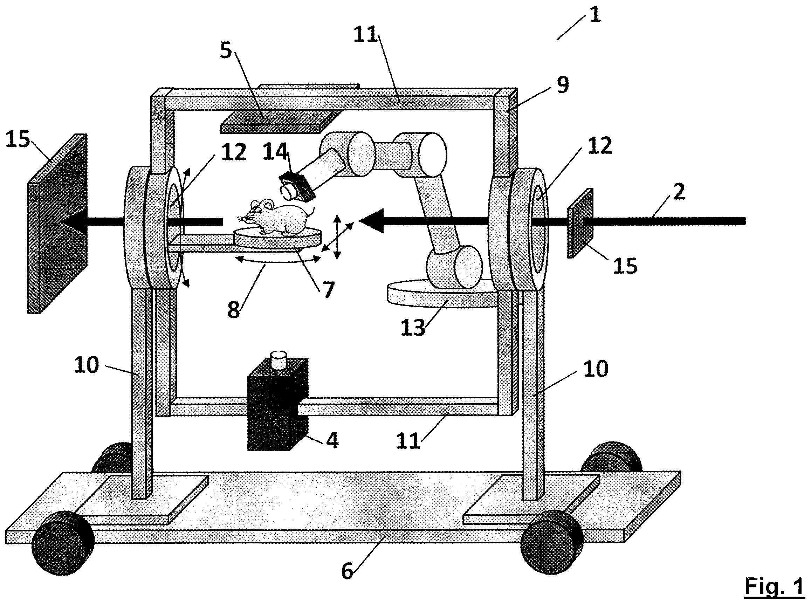

Fig. 1 provides an illustration of the unit **1** for irradiation of the object by the therapeutic beam **2** of particles from the external source **3** not included in the figure along with the concurrent CT scanning of the object using at least one scanning pair consisting of the source **4** of the scanning ionizing radiation and the imaging detector **5** of ionizing radiation arranged on the opposite side. The therapeutic beam **2** of particles can be, for example, a beam of protons that were accelerated in the particle accelerator forming the external source **3** not provided in the figure. A person skilled in the field of radiotherapeutic irradiation will be able to describe a whole group of therapeutic beams **2,** including a group of external sources **3** for the creating thereof. The source **4** of scanning ionizing radiation comprises a powered x-ray tube for the emission of the scanning x-rays; the imaging detector **5** can be, for example, a semiconductor hybrid detector. As in the case of the therapeutic beam **2** of particles, a person skilled in the field of the CT scanning process will be able to create by routine work additional variants of the scanning pair, functionally equivalent to the disclosure provided in the patent application. The object intended for irradiation is in particular a laboratory animal.

The unit **1** comprises the mobile platform **6** for its easy transport to/from the radiotherapy vault **16.** The mobile platform **6** is comprised of a load-bearing structure equipped with travelling wheels and a brake to fix the unit **1** on the spot. The mobile platform **6** supports the adjustable table **7** allowing the object to be positioned with six degrees of freedom. In addition, the mobile platform **6** includes a load-bearing means formed by the rotating support frame **9** illustrated in Fig. 1. The drive of the rotating frame **9** is provided in the form of electric motor not shown in the figure. The load-bearing means, scanning pair and adjustable table **7** limit the free working space **8** for unshielded travel of the therapeutic beam **2** of particles through the unit.

The rotating support frame **9** is fixed using axial bearings between two columns **10** protruding from the mobile platform **6.** The axial bearings have a wide inner ring **12** for unshielded travel of the therapeutic beam **2** of particles into the working space **8** towards the object and further.

In the illustrated example of the embodiment, the rotating support frame **9** has two beams **11,** where one of the beams **11** includes the source **4** of scanning ionizing radiation installed in an adjustable manner and the other beam **11** includes the imaging detector **5** installed in an adjustable manner. The rotating support frame **9** allows the CT scanning process to be executed by the scanning pair. In addition, Fig. 1 illustrates the robotic arm **13** equipped with the source **14** of therapeutic ionizing radiation. The robotic arm **13** can be adjusted in a manner allowing therapeutic irradiation of the object from two sources **3**, **14** of therapeutic radiation to be executed at the same time, including CT scanning during which the rotating support frame **9** is moving along only a part of its trajectory. Figure 1 provides an illustration of a simplified scheme, however, the robotic arm **13** is also supported by the mobile platform **6.** In addition, the mobile platform **6** supports the imaging detectors **15** arranged along the trajectory of the therapeutic beam **2** of particles at the inlet and outlet of the therapeutic beam **2** to/from the free working space **8.**

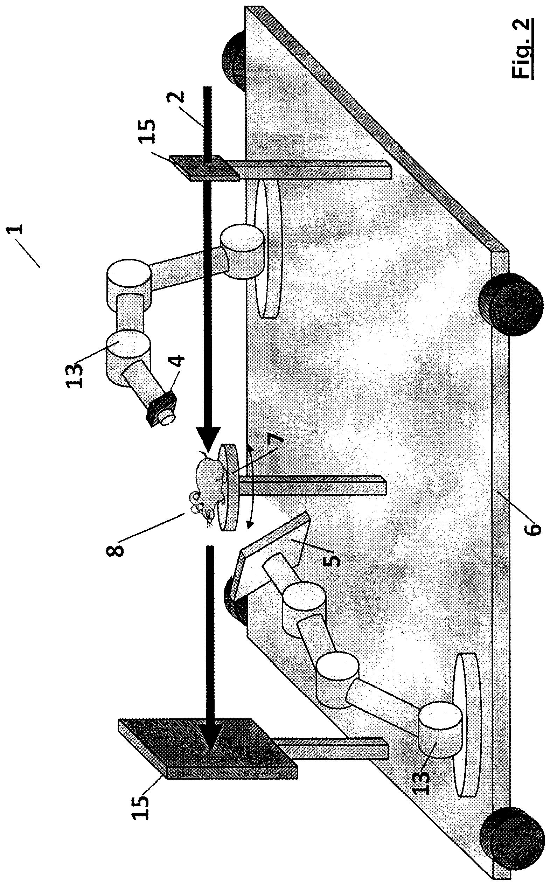

Fig. 2 provides an illustration of the embodiment of the unit **1** with the mobile platform **6** equipped with load-bearing means implemented only by the robotic arms **13.** The illustrated robotic arms **13** support the scanning pair. The unit **1** has the robotic arms **13** equipped with the source **14** of therapeutic ionizing radiation (not shown), adjustable table **7,** and also, for example, detectors **15.** The detectors **15** can be also held by robotic arms **13** to allow changing positions of the detector **15** in respect to a particle beam **2** that is delivered from varying directions by a rotating gantry. The robotic arms **13** allow precise positioning in space.

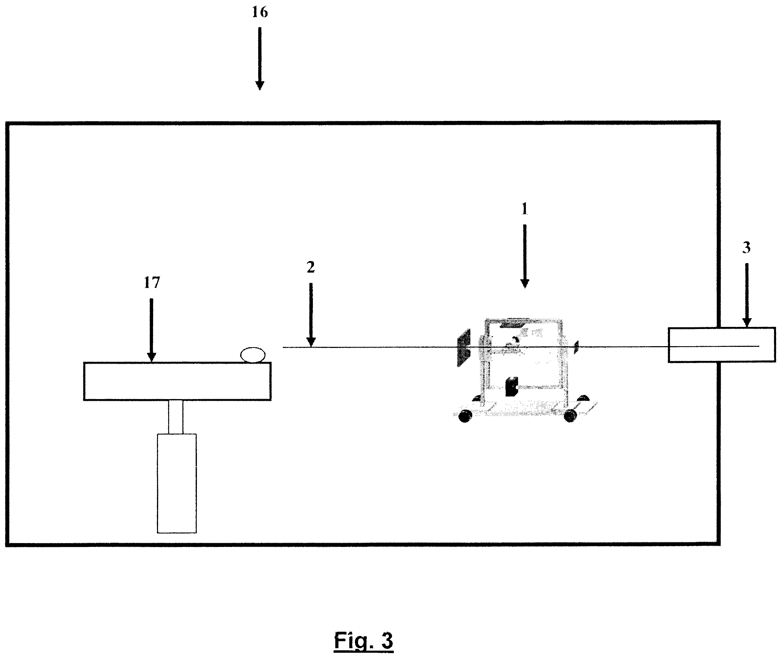

Figure 3 illustrates a diagram of the radiotherapy vault **16** and the position of the unit **1** inside the radiotherapy vault **16.** The therapeutic beam **2** of particles from the external source **3** is supplied to the radiotherapy vault **16.** The interior of the radiotherapy vault **16,** such as the table **17** for patients, does not need to be removed from the radiotherapy vault **16.**

### Industrial Applicability

The unit and equipment for radiotherapeutic preclinical studies according to the invention will be used in scientific research and medicine when performing radiobiology preclinical studies on animals.

### Overview of the positions

- 1: irradiation unit
- 2: therapeutic particle beam
- 3: external source
- 4: scanning ionizing radiation source
- 5: imaging detector
- 6: mobile platform
- 7: adjustable table
- 8: free working space
- 9: rotating support frame
- 10: vertical column
- 11: rotating support frame arm
- 12: inner ring of the axial bearing
- 13: robotic arm
- 14: therapeutic ionizing radiation source
- 15: imaging detector
- 16: radiotherapy vault
- 17: table for patients

## Claims

1. A unit (1) for the irradiation of an object for radiotherapeutic preclinical studies on animals by a therapeutic beam (2) of particles from an external source (3) and simultaneous computed tomography (CT) scanning of the object comprising at least one scanning pair consisting of a source (4) of scanning ionizing radiation and of an imaging detector (5) of ionizing radiation arranged on the opposite side, whereas the unit (1) comprises a mobile platform (6) equipped with an adjustable table (7) to support the irradiated object and equipped with at least one load-bearing means supporting the at least one scanning pair installed in a movable manner with respect to the adjustable table (7), where the load-bearing means, scanning pair and adjustable table (7) delimit a free working space (8) for unshielded travel of the therapeutic beam (2) of particles through the unit (1) **characterized in that** the mobile platform (6) is further equipped with at least one adjustable source (14) of therapeutic ionizing radiation.

2. The unit according to claim 1 **characterized in that** the load-bearing means consists of a rotating support frame (9) fixed using axial bearings between two columns (10) protruding from the mobile platform (6), where the rotating support frame (9) has at least two arms (11) to support the scanning pair, and where the columns (10) and arms (11) of the rotating support frame (9) delimit the free working space (8), and with inner rings (12) of the axial bearings arranged at an inlet of the therapeutic beam (2) of particles to the delimited free working space (8) and at an outlet of the therapeutic beam (2) of particles from the free working space (8) for unshielded travel of the therapeutic beam (2) of particles through the load-bearing means.

3. The unit according to claim 2 **characterized in that** the scanning pair on the arms (11) of the rotating support frame (9) is adjustable.

4. The unit according to claim 1 **characterized in that** the load-bearing means consists of at least one robotic arm (13).

5. The unit according to claim 4 **characterized in that** the at least one adjustable source (14) of therapeutic ionizing radiation is arranged at the robotic arm (13) itself.

6. The unit according to any of claims 1 to 5 **characterized in that** it is equipped with at least one imaging detector (15) of the therapeutic beam (2) of particles.

7. The unit according to claim 6 **characterized in that** it is equipped with two imaging detectors (15) of the therapeutic beam (2) of particles, where the first imaging detector (15) is arranged at an inlet of the therapeutic beam (2) of particles into the delimited free working space (8) and the other imaging detector (15) is arranged at an the outlet of the therapeutic beam (2) of particles from the free working space (8).

8. The unit according to claim 6 when this depends on any of claims 2-5 **characterized in that** the imaging detector (15) is arranged at the rotating support frame (9) or the column (10) or the robotic arm (13).

9. An equipment to conduct preclinical studies using ionising beams on animals inside radiotherapy vaults (16) for clinical radiotherapeutic irradiation of patients with an outlet of the external source (3) of the therapeutic beam (2) of particles installed in the inner space of the vaults **characterized in that** the equipment comprises the unit (1) designed according to any of claims 1 through 8 that is placed in an inner space of the radiotherapy vault (16), where the unit (1) is arranged as to the outlet of the external source (3) for unshielded travel of the therapeutic beam (2) of particles through the free working space (8) delimited by the unit (1).

## Patentansprüche

1. Eine Einheit (1) zur Bestrahlung eines Objekts für radiotherapeutische präklinische Untersuchungen an Tieren mit einem therapeutischen Strahl (2) von Partikeln aus einer externen Quelle (3), gleichzeitig mit einer Computertomographie (CT) zum Scannen des Objekts ausgestattet, mit mindestens einem Abtastpaar bestehend aus einer Quelle (4) zum Scannen der ionisierenden Strahlung und einem Detektor (5) ionisierender Strahlung zur Bilderfassung, der auf der gegenüberliegenden Seite angeordnet ist, wobei die Einheit (1) eine bewegliche Plattform (6) umfasst, ausgestattet mit einem einstellbaren Tisch (7), um das bestrahlte Objekt zu stützen, und ausgerüstet mit mindestens einem tragenden Mittel, das mindestens ein Abtastpaar trägt, das gegenüber dem einstellbaren Tisch (7) beweglich installiert ist , wo das tragende Mittel, das Abtastpaar und der einstellbare Tisch (7) den abschirmungsfreien Arbeitsraum (8) zur Bewegung des therapeutischen Strahls (2) von Partikeln durch die Einheit (1) begrenzen, **dadurchgekennzeic hnet, dass** die bewegliche Plattform (6) ferner mit mindestens einer einstellbaren Quelle (14) therapeutischer ionisierender Strahlung ausgestattet ist.

2. Eine Einheit nach Anspruch 1 **dadurch gekennzeichnet, dass** das tragende Mittel aus einem rotierenden Stützrahmen (9) besteht, fixiert mit zwei Axiallagern zwischen zwei Säulen (10), die aus der beweglichen Plattform (6) herausragen, wobei der rotierende Stützrahmen (9) wenigstens zwei Arme (11) zur Abstützung des Abtastpaares besitzt, wo die Säulen (10) und Arme (11) des rotierenden Stützrahmens (9) den freien Raum für Arbeitsbewegung (8) abgrenzen, mit Innenrings (12) der Axiallager, angeordnet am Eintritt des therapeutischen Strahls (2) von Partikeln zum begrenzten freien Arbeitsraum (8) und am Austritt des therapeutischen Strahls (2) von Partikeln aus dem freien Arbeitsraum (8) für nicht abgeschirmten Durchlauf des therapeutischen Strahls (2) von Partikeln durch das tragende Mittel.

3. Eine Einheit nach Anspruch 2 **dadurch gekennzeichnet, dass** das Abtastpaar auf den Armen (11) des rotierenden Stützrahmens (9) als einstellbar konzipiert ist.

4. Eine Einheit nach Anspruch 1 **dadurch gekennzeichnet, dass** die tragenden Mittel wenigstens einen Roboterarm besitzen (13).

5. Eine Einheit nach Anspruch 4 **dadurch gekennzeichnet, dass** sich auf dem Roboterarm (13) selbst eine einstellbare Quelle (14) therapeutischer ionisierender Strahlung befindet.

6. Eine Einheit nach einem der Ansprüche l bis5 **dadurch gekennzeichnet, d ass** sie wenigstens mit einem Detektor (15) therapeutischen Strahls (2) von Partikeln zur Bilderfassung ausgerüstet ist.

7. Eine Einheit nach Anspruch 6 **dadurch gekennzeichnet, dass** sie mit zwei Detektoren (15) therapeutischen Strahls (2) von Partikeln zur Bilderfassung ausgestattet ist, wobei sich der erste Detektor (15) der Bilderfassung am Eintritt des therapeutischen Strahls (2) von Partikeln zum abgegrenzten Arbeitsraum (8) und der zweite Detektor (15) der Bilderfassung am Austritt des therapeutischen Strahls (2) von Partikeln aus dem freien Arbeitsraum (8) befindet.

8. Eine Einheit nach Anspruch 6, falls dies von einem der Ansprüche 2 bis 5 abhängt, **dadurch gekennzeichnet, dass** der Detektor (15) zur Bilderfassung am rotierenden Stützrahmen (9) oder an der Säule (10) oder am Roboterarm (13) angeordnet ist.

9. Eine Anlage zur Durchführung präklinischer Studien an Tieren unter Verwendung ionisierender Strahlung in Bogenzellen (16) für klinische radiotherapeutische Bestrahlung von Patienten mit dem Austritt einer externen Quelle (3) des therapeutischen Strahls (2) von Partikeln, installiert im inneren Raum der Bogenzelle **dadurchgekennzeic hnet, dass** die Anlage aus einer nach einem der Ansprüche 1 bis 8 konzipierten Einheit (1) besteht, die sich im Innenraum der radiotherapeutischen Bogenzelle (16) befindet, wobei die Einheit (1) in der Richtung zum Austritt des therapeutischen Strahls der externen Quelle (3) für ungeschirmten Durchlauf dieses therapeutischen Strahls (2) von Partikeln durch den von der Einheit (1) definierten freien Arbeitsraum (8) angeordnet ist.

## Revendications

1. Unité (1) pour l'irradiation d'un objet pour des études précliniques radio-thérapeutiques sur des animaux par un faisceau thérapeutique (2) de particules provenant d'une source externe (3) avec une scanographie (CT) pour scanner l'objet comprenant au moins une paire de scanographie constitué d'une source (4) de rayonnement ionisant de scanographie et d'un capteur d'image (5) de rayonnement ionisant disposé sur le côté opposé, tandis que l'unité (1) comprend une plate-forme mobile (6) équipée d'une table réglable (7) pour supporter l'objet irradié et équipée d'au moins un moyen porteur supportant d'au moins une paire de scanographie installée par rapport à la table réglable (7) de manière mobile, où les moyens porteurs, la paire de scanographie et la table réglable (7) délimitent l'espace de travail libre (8) pour le déplacement non blindé du faisceau thérapeutique (2) de particules à travers l'unité (1) **caractérisé en ce qu e** la plate-forme mobile (6) est en outre équipée d'au moins une source réglable (14) de rayonnement ionisant thérapeutique.

2. Unité selon la revendication 1, **caractérisée en ce que** le moyen porteur est constitué d'un cadre de support rotatif (9) fixé à l'aide de paliers axiaux entre deux colonnes (10) faisant saillie de la plate-forme mobile (6), où le cadre de support rotatif (9) comporte au moins deux bras (11) pour supporter la paire de scanographie, et où les colonnes (10) et les bras (11) du cadre de support rotatif (9) délimitent l'espace de travail libre (8), ainsi qu'avec des bagues intérieures (12) des paliers axiaux disposées à l'entrée du faisceau thérapeutique (2) de particules vers l'espace de travail libre délimité (8) et à la sortie du faisceau thérapeutique (2) de particules de l'espace de travail libre (8) pour le déplacement non blindé du faisceau thérapeutique (2) de particules à travers des moyens porteurs.

3. Unité selon la revendication 2 **caractérisée en ce que** la paire de scanographie située sur les bras (11) du cadre de support rotatif (9) est ajustable.

4. Unité selon la revendication 1 **caractérisée en ce que** les moyens porteurs comportent au moins un bras robotique (13).

5. Unité selon la revendication 4 **caractérisé**e **en ce qu**'au moins une source ajustable (14) de rayonnement ionisant thérapeutique est disposée au niveau du bras robotique (13) lui-même.

6. Unité selon l'une des revendications 1 à 5 **caractérisée en ce qu**'elle est équipée d'au moins un détecteur d'image (15) du faisceau thérapeutique (2) de particules.

7. Unité selon la revendication 6 **caractérisée en ce qu**'elle est équipée de deux détecteurs d'image (15) du faisceau thérapeutique (2) de particules où le premier détecteur d'image (15) est disposé à l'entrée du faisceau thérapeutique (2) de particules dans l'espace de travail libre délimité (8) et l'autre détecteur d'image (15) est disposé à la sortie du faisceau thérapeutique (2) ) de particules de l'espace de travail libre (8).

8. Unité selon la revendication 6 si cela dépend de l'une des revendications 2 à 5 c **aractérisé**e **en ce que** le détecteur d'image (15) est installé sur le cadre de support rotatif (9) ou sur la colonne (10) ou sur le bras robotique (13).

9. Un équipement pour effectuer des études précliniques utilisant des faisceaux ionisants sur des animaux à l'intérieur de voûtes de radiothérapie (16) pour l'irradiation radio-thérapeutique clinique des patients par la sortie de la source externe (3) du faisceau thérapeutique (2) de particules, installé dans l'espace intérieur des voûtes **caractérisée en ce que** cet équipements contient une unité (1) conçue selon l'une des revendications 1 à 8, placé dans un espace intérieur de la voûte de radiothérapie (16), où l'unité (1) est disposé à la sortie de la source externe (3) pour le déplacement non blindé du faisceau thérapeutique (2) de particules à travers l'espace de travail libre (8), délimité par l'unité (1).
